# EUROPÄISCHE PATENTSCHRIFT

(11) **EP 1 891 907 B1**
(45) Veröffentlichungstag und Bekanntmachung des Hinweises auf die Patenterteilung: **13.10.2010**
(21) Anmeldenummer: 07015075.0
(22) Anmeldetag: 01.08.2007
(51) Int. Cl.: A61B 18/14

(54) **Vorrichtung zur Resektion und/oder Ablation von organischem Gewebe mittels Hochfrequenzstrom sowie Resektoskop**
Device for resection and or ablation of organic tissue using high frequency electricity and a resectoscope
Dispositif destiné à la résection et/ou l'ablation de tissus organiques à l'aide d'une énergie haute fréquence tout comme résectoscope

(30) Priorität: 21.08.2006 DE 102006039696
(43) Veröffentlichungstag der Anmeldung: 27.02.2008
(73) Patentinhaber: Hamou, Jacques, 75016 Paris (FR)
(72) Erfinder: Hamou, Jacques, 75016 Paris (FR)
(74) Vertreter: Heuckeroth, Volker

(56) Entgegenhaltungen:
- WO-A-2006/048199
- DE-C- 848 233
- US-A1- 2003 130 653
- US-A1- 2004 138 654
- US-A1- 2006 089 639

## Beschreibung

Die Erfindung betrifft eine Vorrichtung zur Resektion und/oder Ablation von organischem Gewebe mittels Hochfrequenzstrom gemäß dem Oberbegriff des Anspruchs 1.

Die Erfindung betrifft ferner ein Resektoskop zum Abtragen von organischem Gewebe mittels Hochfrequenzstrom, mit einem Schaft, in dem eine Vorrichtung der zuvor genannten Art angeordnet ist.

Eine derartige Vorrichtung ist aus der WO 2006/048199 und FR 2877207 bekannt.

Solch eine Vorrichtung bzw. ein Resektoskop wird in der Hochfrequenzchirurgie zur Resektion und Ablation von organischem Gewebe verwendet. Insbesondere kann die Vorrichtung in der Hysteroskopie bzw. in der Urologie eingesetzt werden, um Fibrome, Polypen und Gewebe des Endometriums bzw. prostatische Adenome zu entfernen.

Eine aus US 2004/0064139 A1 bekannte Vorrichtung weist einen Schlingenträger, eine elektrisch leitende und mit Hochfrequenzstrom beaufschlagbare Schlinge sowie ein Verbindungselement in Form zweier gerader Stränge zwischen dem Schlingenträger und der Schlinge auf. Die Schlinge erstreckt sich entweder in einer Ebene quer zu einer Längsachse des Schlingenträgers oder in einer Ebene, die die Längsachse des Schlingenträgers enthält.

Um organisches Gewebe mittels der im Resektoskop aufgenommenen Vorrichtung zu entfernen, wird diese vom Arzt entlang der Längsrichtung des Schlingenträgers bewegt. Die mit Hochfrequenzspannung beaufschlagbare Schlinge trennt das zu entfernende Gewebe somit in einem ziehenden Schnitt ab. Gleichzeitig wird die Schnittfläche mittels der Schlinge verödet, um möglicherweise auftretende Blutungen zu stillen. Die abgelösten Gewebestücke müssen in einem weiteren Arbeitsschritt aus dem Operationsgebiet entnommen werden.

Alle in dem zuvor genannten Dokument US 2004/0064139 A1 beschriebenen Ausgestaltungen der Anordnung aus Schlingenträger, Verbindungselement und Schlinge eigenen sich nachteiligerweise nur zum Schneiden von Gewebe mittels eines ziehenden Schnittes, bei dem also die Schlinge in Längsrichtung des Schlingenträgers bewegt wird.

Aus der oben genannten WO 2006/048199 A1 ist eine Vorrichtung der eingangs genannten Art bekannt, die einen Schlingenträger in Form eines Hohlrohres, eine distalseitig des Schlingenträgers angeordnete Schlinge und ein als Doppelwendel ausgebildetes Verbindungselement zwischen der Schlinge und dem Schlingenträger aufweist. Die Schlinge ist halbkreisförmig ausgebildet und gegen eine Längsachse des Schlingenträgers geringfügig geneigt und mit Hochfrequenzspannung beaufschlagbar. Mit dieser Vorrichtung wird Gewebe geschnitten, indem die Schlinge und die Doppelwendel in Rotation um die Längsachse des Hohlrohrs versetzt wird. Die mitrotierende Doppelwendel soll auch zum Transport von geschnittenem organischen Gewebe nach proximal dienen, wobei das Gewebe bei seiner Einführung in das Rohr zusätzlich nach proximal abgesaugt werden kann.

Je nach der Topologie des zu schneidenden Gewebes liegt die halbkreisförmige Schlinge zu Beginn des Schnitts mehr oder weniger linienförmig an dem Gewebe an, wobei es auch vorkommen kann, dass einzelne Schlingenabschnitte das Gewebe berühren, andere jedoch nicht. Dies ist der Fall, wenn das zu schneidende Gewebe konvexe und konkave Bereiche aufweist. Mit anderen Worten gibt es an der Schlinge keine definierte Stelle, an der der Schnitt beginnt. Durch das zumindest abschnittsweise linienförmige Anliegen der Schlinge an dem zu schneidenden Gewebe ist auch die Schneidwirkung der bekannten Vorrichtung nicht optimal. Das Gleiche gilt auch für die aus US 2004/0064139 A1 bekannte Vorrichtung, bei der die Schlinge aufgrund ihrer Symmetrie keinen definierten Anschnitt ermöglicht.

Aus US 2003/0130653 A1 ist eine weitere Vorrichtung zur Resektion und/oder Ablation von Gewebe mittels Hochfrequenzstrom bekannt. Die Vorrichtung weist einen lang erstreckten Körper auf, wobei zumindest ein Arm mit dem distalen Ende des Körpers verbunden ist, und wobei eine Elektrode mit dem zumindest einen Arm verbunden ist. Die Elektrode umfasst eine obere Oberfläche und eine untere Oberfläche. Die untere Oberfläche ist im Wesentlichen konvex ausgebildet und definiert einen Krümmungsradius relativ zu einer im Wesentlichen senkrecht zur Längsachse verlaufenden Achse. In diesem Dokument werden verschiedene Elektrodengeometrien vorgeschlagen, darunter auch eine Schlingenelektrode, wie sie aus dem oben genannten Dokument US 2004/0064139 A1 bekannt ist. Mit diesen Elektrodengeometrien werden ziehende Schnitte ausgeführt. Der Elektrodenträger ist nicht um seine Längsachse drehbar.

Ein weiteres elektrochirurgisches Werkzeug mit einer Schlingenelektrode ist aus US 2006/0089639 A1 bekannt. Diese Schlingenelektrode ist an einer Welle befestigt, die um eine Achse drehbar ist, die senkrecht zur Längsachse des Werkzeugschaftes steht.

US 2004/0138654 offenbart ein elektrochirurgisches Instrument, das als Schneidwerkzeug eine Schlingenelektrode aufweist, die sich wie in US 2004/0064139 A1 senkrecht zum Schlingenträger erstreckt. Der Schlingenträger ist nicht um seine Längsachse drehbar.

Schließlich offenbart DE 848 233 C eine Elektrode zum elektrischen Schneiden in menschlichen Körperhöhlen, die als Schlinge ausgebildet ist, mit der Schneidbewegungen in Richtung der Längsachse ausgeführt werden. Der Schneiddraht, der die Elektrode bildet, weist zwei Enden auf, die in Längsrichtung des Elektrodenträgers voneinander beabstandet am Elektrodenträger befestigt sind. Der Schneiddraht liegt dabei in einer Ebene, die die Längsachse des Elektrodenträgers einschließt. Der Schneiddraht erstreckt sich somit in Längsrichtung des Elektrodenträgers, wodurch der Elektrodenträger nicht gegabelt ausgeführt werden muss, wie dies bspw. bei der Vorrichtung gemäß US 2004/0064139 A1 der Fall ist.

Es ist eine Aufgabe der vorliegenden Erfindung, eine Vorrichtung der eingangs genannten Art zu schaffen, die eine gezielte effektivere Gewebeabtragung erlaubt.

Erfindungsgemäß wird die Aufgabe hinsichtlich der eingangs genannten Vorrichtung dadurch gelöst, dass ein Schlingenendabschnitt der Schlinge und ein distaler Verbindungselementabschnitt des Verbindungselements eine keilförmige Schneide bilden, indem der Schlingenendabschnitt und der distale Verbindungselementabschnitt unter einem Winkel von weniger als 90° aufeinander zulaufen, dass die keilförmige Schneide in die Drehrichtung des Schlingenträgers weist, und dass die keilförmige Schneide geringfügig nach distal weist, so dass sich die keilförmige Schneide aufgrund der geringfügigen Neigung nach distal wie eine Schraube in das Gewebe hineinzieht.

Die erfindungsgemäße Vorrichtung weist demnach an ihrem distalen Ende eine keilförmige Schneide auf. "Keilförmig" bedeutet hier, dass der distale Verbindungselementabschnitt und der Schlingenendabschnitt unter einem spitzen Winkel, d.h. einem Winkel unter 90°, aufeinander zulaufen. Diese Verbindung ist vorzugsweise einstückig. Die keilförmige Schneide eignet sich insbesondere für eine Ausgestaltung der Vorrichtung, bei der die Schlinge zum Schneiden in Rotation versetzt wird. Die keilförmige Schneide bildet eine definierte punktförmige Anlage des wirksamen Schneidbereichs der Schlinge an dem Gewebe. Durch die keilförmige Ausgestaltung der Schneide kann diese wirksam in das zu schneidende Gewebe eindringen und dabei gleichzeitig das abzutrennende Gewebe vom übrigen Gewebe ablösen bzw. abheben. Die erfindungsgemäße Vorrichtung ermöglicht vorteilhafterweise ein Resizieren und Koagulieren von sowohl frontal vor der Schlinge befindlichem Gewebe, als auch von solchem, das sich seitlich des Verbindungselements bzw. der Schlinge befindet. Das frontal befindliche Gewebe wird durch den Schlingenendabschnitt und den übrigen Bereich der Schlinge abgetragen, während das seitlich befindliche Gewebe zumindest durch den distalen Verbindungselementabschnitt abgetragen werden kann.

Der Schlingenträger ist um seine Längsachse rotierbar und ein Endbereich der keilförmigen Schneide weist in Drehrichtung des Schlingenträgers.

Die keilförmige Schneide bildet demnach das vorauseilende Ende der Schlinge, wenn der Schlingenträger um seine Längsachse in Rotation versetzt wird. Die keilförmige Schneide kann somit effektiv in das Gewebe zum Abtrennen desselben eindringen. Die keilförmige Ausgestaltung der Schneide trennt dabei das Gewebe im Zusammenwirken mit dem übrigen nachlaufenden Abschnitt der Schlinge effektiv ab.

Die keilförmige Schneide weist geringfügig nach distal.

Diese Maßnahme hat den Vorteil, dass sich die keilförmige Schneide aufgrund der geringfügigen Neigung nach distal wie eine Schraube in das Gewebe hinein zieht, wodurch beim Schneiden kein oder kein wesentlicher Druck in Vorschubrichtung ausgeübt werden muss.

In einer bevorzugten Ausgestaltung erstreckt sich die Schlinge in einer Ebene quer zu einer Längsachse des Schlingenträgers.

Diese Maßnahme hat den Vorteil, dass im Fall, dass die Schlinge über den Schlingenträger in Rotation versetzbar ist, die Schlinge, die üblicherweise aus einem dünnen Draht gebildet ist, wie eine Sichel in Längserstreckung des Drahtes durch das Gewebe hindurch schneidet. Dadurch erfährt die Schlinge beim Schneiden keinen erhöhten mechanischen Widerstand und unterliegt weniger der Gefahr eines Verbiegens oder gar Brechens.

In einer weiteren bevorzugten Ausgestaltung ist das Verbindungselement schraubenlinienförmig ausgebildet.

Diese Maßnahme hat den Vorteil, dass die Vorrichtung distalseitig eine kompakte Bauweise aufweist, und dass die Stabilität der Vorrichtung erhöht ist. Ferner kann seitlich befindliches Gewebe zusätzlich mit dem am Gewebe anliegenden Bereich der Windungen in der Art einer korkenzieherartigen Sichel kontinuierlich abgetragen werden. Dies ist insbesondere bei einem Vergleich zu einer geraden Ausgestaltung des Verbindungselements von Vorteil, da der operative Eingriff schneller durchgeführt werden kann. Solange das abgetrennte Gewebe noch nicht vollständig abgetrennt ist, bewirkt die schraubenförmige Ausgestaltung, dass das Gewebe aufgrund einer Relativbewegung zum Verbindungselement nach proximal gefördert wird.

In einer weiteren bevorzugten Ausgestaltung weist das Verbindungselement einen ersten Strang und einen zweiten Strang auf, wobei ein distaler Endbereich des ersten Strangs mit dem Schlingenendabschnitt die keilförmige Schneide bildet, und ein distaler Endbereich des zweiten Strangs mit dem dem Schlingenendabschnitt entgegengesetzten Schlingenende der Schlinge verbunden ist.

Diese Maßnahme hat den Vorteil, dass die Stabilität des Verbindungselements gegenüber äußeren Krafteinwirkungen beim Schneidvorgang erhöht ist, da die Schlinge nicht nur im Bereich der keilförmigen Schneide mit dem Verbindungselement verbunden ist, sondern auch an einem zweiten Ende, so dass sie insbesondere gegenüber Torsionskräften, die beim rotierenden Schneiden mit der Schlinge auftreten können, verwindungssteif ist.

In einer weiteren bevorzugten Ausgestaltung sind der erste Strang und der zweite Strang des Verbindungselements gegenläufig schraubenlinienförmig gewunden.

Diese Maßnahme trägt noch zur besseren Verwindungssteifigkeit der Schlinge gegenüber dem Schlingenträger bei. Somit kann auch festes Gewebe sicher abgetragen werden.

In einer weiteren bevorzugten Ausgestaltung ist die Schlinge etwa halbkreisförmig ausgebildet.

Diese an sich bekannte Maßnahme hat in Verbindung mit der Ausbildung einer keilförmigen Schneide an dem einen Schlingenendabschnitt den Vorteil einer besonders effizienten Schneidwirkung der Schlinge, da der weiterschneidende Bereich der Schlinge ausreichend groß ist.

In einer weiteren bevorzugten Ausgestaltung weist die Schlinge innenseitig und/oder außenseitig eine Schneidkante auf.

Diese Maßnahme hat den Vorteil, dass die Schlinge nicht nur im Bereich der keilförmigen Schneide gewebeabtragend wirkt, sondern auch in ihrem übrigen Bereich, wodurch die Schneideffizienz der erfindungsgemäßen Vorrichtung weiter verbessert ist.

In einer weiteren bevorzugten Ausgestaltung weist das Verbindungselement zumindest im Bereich des distalen Verbindungselementabschnitts innenseitig und/oder außenseitig eine Schneidkante auf.

Diese Maßnahme hat den Vorteil, dass auch seitlich befindliches Gewebe effektiv abgetragen werden kann. Während der Drehbewegung des Schlingenträgers greift die vorzugsweise außen- und innenseitig angeordnete Schneidkante des Verbindungselementbschnitts in das abzutragende Gewebe ein und durchtrennt dieses in einem scharfen Schnitt.

In einer weiteren bevorzugten Ausgestaltung ist der Schlingenträger als Hohlrohr ausgebildet.

Diese Maßnahme hat den Vorteil, dass mittels der Schlinge und ggf. des Verbindungselements abgetrenntes Gewebe durch das Hohlrohr aus dem Körper abgeführt werden kann, beispielsweise indem an das proximale Ende des Hohlrohrs ein Saugstrom angelegt wird. Auf diese Weise wird das abgetrennte Gewebe permanent aus dem Körper abgeführt, ohne dass das abgetrennte Gewebe mittels eines zusätzlichen Instruments entnommen werden muss.

Das erfindungsgemäße Resektoskop weist eine Vorrichtung gemäß einer oder mehrerer der zuvor genannten Ausgestaltungen auf.

Weitere Vorteile und Merkmale ergeben sich aus der nachfolgenden Beschreibung und der beigefügten Zeichnung.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Die Erfindung wird nachfolgend anhand ausgewählter Ausführungsbeispiele im Zusammenhang mit der beiliegenden Zeichnung näher beschrieben und erläutert. Es zeigen:
- Fig. 1: eine perspektivische Darstellung einer Vorrichtung zur Resektion und/oder Ablation von organischem Gewebe mittels Hochfrequenzstrom;
- Fig. 2A: eine Vorderansicht der Vorrichtung in Fig. 1;
- Fig. 2B - 2D: Seitenansichten der Vorrichtung in Fig. 1 im Bereich ihres distalen Endes;
- Fig. 3A - 3C: perspektivische Darstellungen des Verbindungselements und der Schlinge der Vorrichtung in Fig. 1 in drei Drehstellungen; und
- Fig. 4: eine Seitenansicht eines Resektoskops, in dem die Vorrichtung in Fig. 1 aufgenommen ist.

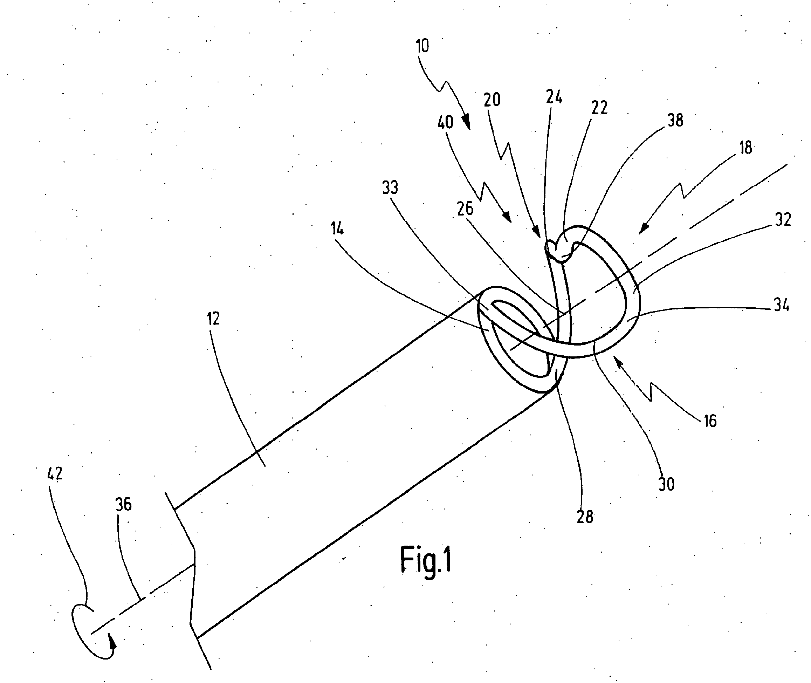

In Fig. 1 ist eine mit dem allgemeinen Bezugszeichen 10 versehene Vorrichtung zur Resektion und/oder Ablation von organischem Gewebe mittels Hochfrequenzstrom dargestellt.

Die Vorrichtung 10 kann bspw. in der Hysteroskopie bzw. der Urologie verwendet werden, um Fibrome, Polypen und Endrometriumsgewebe bzw. prostatische Adenome zu entfernen.

Die Vorrichtung 10 weist einen Schlingenträger 12 auf, an dessen distalem Ende 14 ein Verbindungselement 16 sowie eine mit Hochfrequenzspannung beaufschlagbare Schlinge 18 angeordnet sind.

Der Schlingenträger 12 ist als zylinderförmiges Hohlrohr ausgebildet, wobei das Verbindungselement 16 mit einer ringförmigen Stirnfläche des distalen Endes 14 des Hohlrohrs verbunden ist.

Ein distaler Verbindungselementabschnitt 20 des Verbindungselements 16 ist keilförmig mit einem Schlingenendabschnitt 22 der Schlinge 18 verbunden, d.h. der Verbindungselementabschnitt 20 und der Schlingenendabschnitt laufen unter einem spitzen Winkel von weniger als 90° zusammen.

Zumindest der distale Verbindungselementabschnitt 20 ist wie die Schlinge 18 stromführend ausgebildet und außenseitig nicht isoliert.

In dem dargestellten Ausführungsbeispiel stellt der Verbindungselementabschnitt 20 einen distalen Endbereich 24 eines ersten Strangs 26 des Verbindungselements 16 dar, wobei der erste Strang 26 an einem proximalen Ende 28 mit dem distalen Ende 14 des Schlingenträgers 12 verbunden ist. Das Verbindungselement 16 weist ferner einen zweiten Strang 30 auf, der zwischen dem distalen Ende 14 des Schlingenträgers 12 und einem Schlingenende 32 der Schlinge 18 angeordnet ist. Hierzu ist ein proximales Ende 33 des zweiten Strangs 30 dem distalen Ende 14 des Schlingenträgers 12 an einer der Verbindungsstelle des ersten Strangs 26 mit dem distalen Ende 14 des Schlingenträgers 12 um etwa 180° versetzten Position verbunden, und ein distaler Endbereich 34 des zweiten Strangs 30 ist mit dem Schlingenende 32 verbunden.

Der Schlingenendabschnitt 22 und der distale Endbereich 24 des ersten Strangs 26 sind spitzwinklig, d.h. unter einem kleinen Winkel von weniger als 90° miteinander verbunden.

Die Schlinge 18 und die Stränge 26, 30 sind drahtförmig ausgebildet.

Das Verbindungselement 16 ist schraubenlinienförmig ausgebildet, wobei beide Stränge 26, 30 gegenläufig schraubenlinienförmig gewunden sind, d.h. sie weisen jeweils in eine Längsrichtung 36 des Schlingenträgers 12 gesehen um eine halbe Ganghöhe versetzte gegensinnige Windungen auf. Jeder Strang 26, 30 weist eine halbe Windung auf. Die beiden Stränge 26, 30 des Verbindungselements 16 können auch mehrere Windungen aufweisen.

Die Schlinge 18 erstreckt sich in einer Ebene quer zur Längsachse 36 des Schlingenträgers 12. In dem gezeigten Ausführungsbeispiel erstreckt sich die Schlinge 18 in der Ebene etwa senkrecht zu der Längsachse 36 des Schlingenträgers 12. Eine Ecke 38 im Bereich der Verbindung des Verbindungselementabschnitts 20 mit dem Schlingenendabschnitt 22 bildet eine keilförmige Schneide 40, die in der Ebene der Schlinge 18 liegt. Die keilförmige Schneide 40 weist geringfügig nach distal, wobei hierzu auch die Ebene, in der sich die Schlinge erstreckt, von einer senkrechten Anordnung bezüglich der Längsachse 36 des Schlingenträgers 12 abweichen kann. Die keilförmige Schneide 40 kann, wie hier dargestellt, abgerundet oder aber spitz zulaufend ausgebildet sein.

Die Schlinge 18 ist etwa halbkreisförmig ausgebildet. Sie kann ebenfalls als Ellipsenbogen oder Kreisbogen mit größerer oder kleinerer Erstreckung als über etwa 180° ausgebildet sein.

Die Schlinge 18 kann innenseitig und/oder außenseitig eine Schneidkante aufweisen oder als Schneidmesser ausgebildet sein. Die Schneidkante bzw. das Schneidmesser können nach innen gerichtet sein. Ferner kann das Verbindungselement 16 zumindest im Bereich des Verbindungselementabschnitts 20 ebenfalls innenseitig und/oder außenseitig eine Schneidkante zum Resezieren von Gewebe aufweisen oder als Schneidmesser ausgebildet sein. Die Schneidkante bzw. das Schneidmesser können sich innenseitig über die gesamte Länge der beiden Stränge 26, 30 erstrecken, wobei die Schneidkante bzw. das Schneidmesser an jeder Position der Stränge tangential ausgerichtet ist.

Die Schlinge 18 und das Verbindungselement 16 können aus einem steifem Draht, insbesondere einem Stahldraht, gebildet sein. Um die Schneidwirkung der Vorrichtung 10 zu verbessern und um eine gleichzeitige Koagulation der Vorrichtung 10 zu erreichen, sind die Schlinge 18 und das Verbindungselement 16 mit Hochfrequenzspannung beaufschlagbar und elektrisch leitend ausgebildet. Hierzu ist das Verbindungselement 16 proximalseitig elektrisch kontaktiert.

Der Schlingenträger 12 ist um seine Längsachse 36 in eine Drehrichtung entlang eines Pfeils 42 rotierbar, so dass die keilförmige Schneide 40 in die Drehrichtung des Schlingenträgers 12 weist. Bei Rotation der Vorrichtung 10 läuft die keilförmige Schneide 40 voraus und bewirkt den Anschnitt, während der Weiterschnitt von dem übrigen, nachlaufenden Abschnitt der Schlinge 18 bewirkt wird. Ferner kann seitlich des Verbindungselements befindliches Gewebe mit den beiden Strängen 26, 30 abgetragen werden.

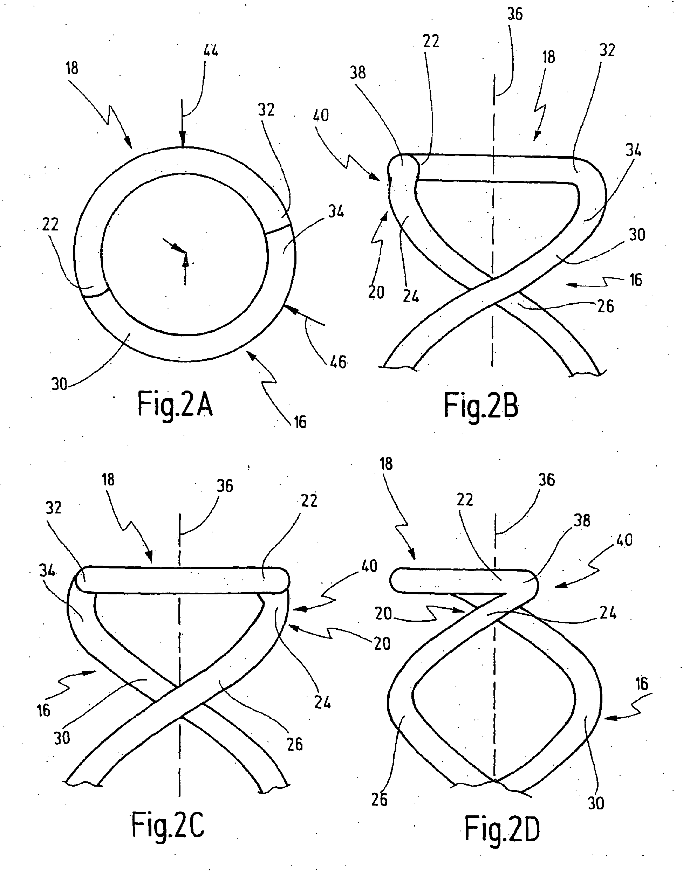

Fig. 2A zeigt eine Draufsicht auf das Verbindungselement 16 und die Schlinge 18 in Fig. 1. Die halbkreisförmige Schlinge 18 liegt in der Zeichenebene, da sie sich in der Ebene etwa senkrecht zur Längsachse 36 des Schlingenträgers 12 erstreckt. Die halbkreisförmige Schlinge 16 weist einen Außenradius 44 auf, der etwa einem Außenradius 46 des zylinderförmigen Verbindungselements 16, d.h. der beiden schraubenlinienförmigen Stränge 26, 30, entspricht. Ein Außenradius des Schlingenträgers 12 kann ebenfalls gleich oder geringfügig kleiner als der Außenradius 44, 46 ausgebildet sein, so dass die Vorrichtung 10, insbesondere das Verbindungselement 16, eine Gewebeoberfläche gleichmäßig abtragen kann.

Fig. 2B - 2D zeigen Seitenansichten des Verbindungselements 16 und der Schlinge 18 in Fig. 1, wobei Fig. 2B eine Ansicht auf die Schlingeninnenseite, Fig. 2C auf die Schlingenaußenseite und Fig. 2D auf den Schlingenendabschnitt 22 und die keilförmige Schneide 40 ist.

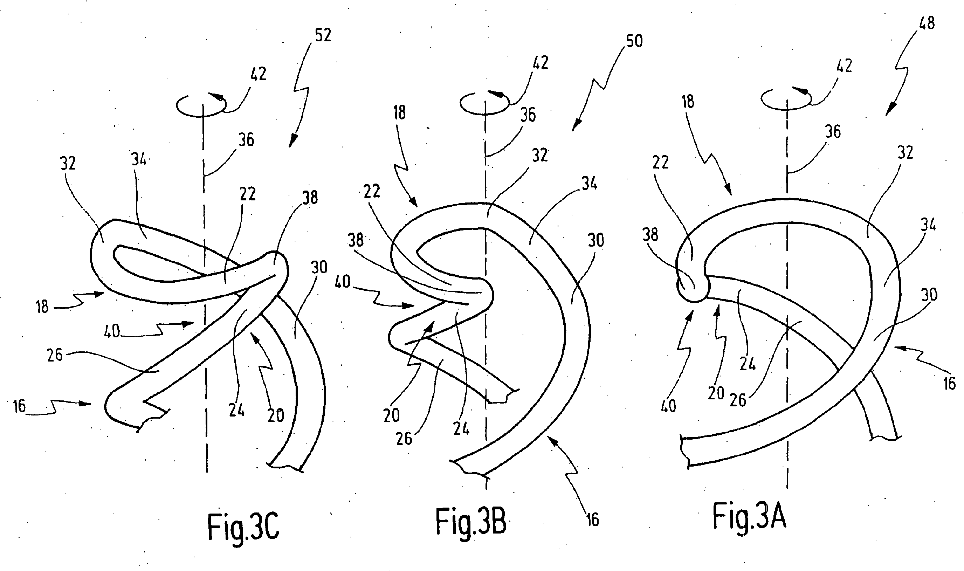

Fig. 3A - 3C zeigen drei Drehstellungen 48-52 des Verbindungselements 16 und der Schlinge 18 in Fig. 1. In der ersten Drehstellung 48 ist eine perspektivische Sicht auf die Schlingeninnenseite dargestellt und die keilförmige Schneide 40 weist aus der Zeichenebene heraus. In der zweiten Drehstellung 50 sind die Schlinge 18 und das Verbindungselement 16 um etwa 90° um die Längsachse 36 des Schlingenträgers 12 entlang der Drehrichtung des Pfeils 42 weitergedreht. In einer dritten Drehstellung 52 sind die Schlinge 18 und das Verbindungselement 16 entlang der Drehrichtung des Pfeils 42 nochmals um etwa 60° weitergedreht.

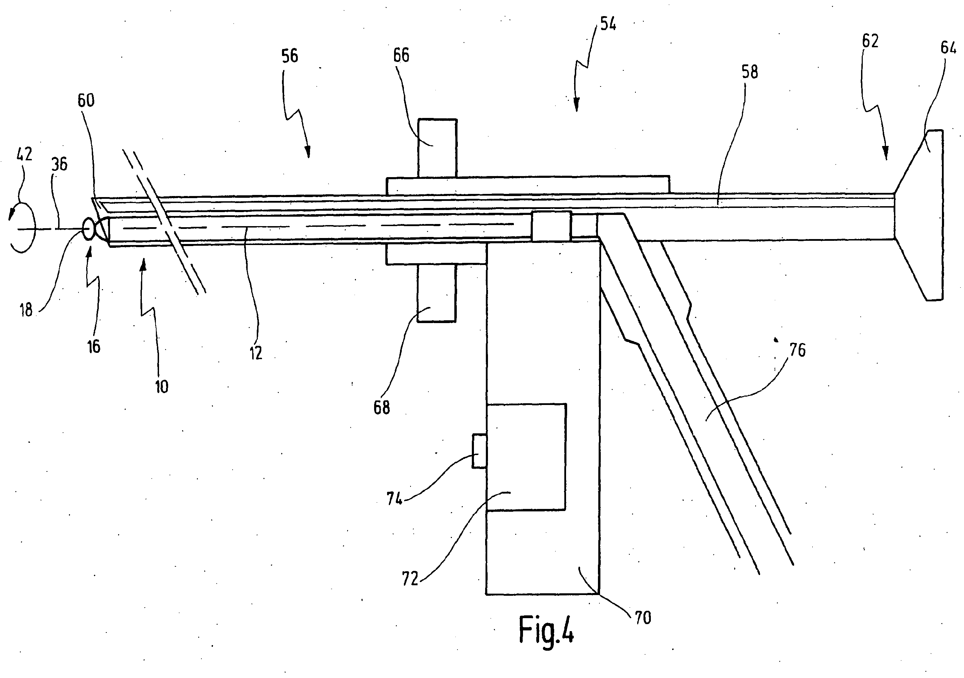

Fig. 4 zeigt ein Resektoskop 54, in dem die Vorrichtung 10 aufgenommen ist. Die Vorrichtung 10 ist in einem Schaft 56 des Resektoskops 54 angeordnet, der als Hohlrohr ausgebildet ist. Ferner weist der Schaft 56 ein Endoskop 58 auf, das sich innerhalb des Schafts 56 von einem distalen Ende 60 des Resektoskops bis zu einem Okular 62 an einem proximalen Ende 64 des Resektoskops 54 erstreckt. Am Schaft 56 des Resektoskops 54 sind Anschlüsse 66, 68 für Spül- und Saugleitungen vorgesehen. Die Anschlüsse 66, 68 dienen zum Zuführen einer Spülflüssigkeit in das Behandlungsgebiet sowie zum Abführen von Flüssigkeit aus dem Behandlungsgebiet. Ferner weist das Resektoskop 54 einen Handgriff 70 auf, in dem ein Motor 72 zur Erzeugung der Drehbewegung der Vorrichtung 10 aufgenommen ist. Der Motor 72 ist manuell mittels eines Schalters 74, hier schematisch als Druckknopf dargestellt, betätigbar. Die Drehgeschwindigkeit des Schlingenträgers 12, d.h. der Vorrichtung 10, kann variabel eingestellt werden.

Eine Saugleitung 76, an der ein Saugstrom anlegbar ist, ist mit dem hohlrohrförmigen Schlingenträger 12 verbunden, so dass abgetrenntes Gewebe über den Schlingenträger 12 und die Saugleitung 76 abgesaugt werden kann.

Die Schlinge 18 und das Verbindungselement 16 ragen zumindest teilweise aus dem Schaft 56 des Resektoskops 54 heraus. Die schraubenlinienförmigen Stränge 26, 30 weisen jeweils eine halbe Windung auf, so dass die Schlinge 18 nur geringfügig vom Ende des Schafts 56 des Resektoskops 54 beabstandet ist. Ferner ist die Schlinge 18 und das Verbindungselement 16 stets innerhalb eines Sichtbereichs des Endoskops 58 angeordnet.

## Patentansprüche

1. Vorrichtung zur Resektion und/oder Ablation von organischem Gewebe mittels Hochfrequenzstrom, mit einem Schlingenträger (12), mit einer mit Hochfrequenzspannung beaufschlagbaren Schlinge (18), die an einem distalen Ende (14) des Schlingenträgers (12) angeordnet ist, und mit einem Verbindungselement (16) zwischen der Schlinge (18) und dem Schlingenträger (12), wobei der Schlingenträger (12) um seine Längsachse (36) in eine Drehrichtung rotierbar ist, wobei ein Schlingenendabschnitt (22) der Schlinge (18) und ein distaler Verbindungselementabschnitt (20) des Verbindungselements (16) eine keilförmige Schneide (40) bilden, indem der Schlingenendabschnitt (22) und der distale Verbindungselementabschnitt (20) unter einem Winkel von weniger als 90° aufeinander zulaufen, und die keilförmige Schneide (40) in die Drehrichtung des Schlingenträgers (12) weist, **dadurch gekennzeichnet, dass** die keilförmige Schneide (40) geringfügig nach distal weist, so dass sich die keilförmige Schneide aufgrund der geringfügigen Neigung nach distal wie eine Schraube in das Gewebe hineinzieht.

2. Vorrichtung nach Anspruch 1, **dadurch gekennzeichnet, dass** sich die Schlinge (18) in einer Ebene etwa senkrecht zu einer Längsachse (36) des Schlingenträgers (12) erstreckt.

3. Vorrichtung nach Anspruch 1 oder 2, **dadurch gekennzeichnet, dass** das Verbindungselement (16) schraubenlinienförmig ausgebildet ist.

4. Vorrichtung nach einem der Ansprüche 1 bis 3, **dadurch gekennzeichnet, dass** das Verbindungselement (16) einen ersten Strang (26) und einen zweiten Strang (30) aufweist, wobei ein distaler Endbereich (24) des ersten Strangs (26) mit dem Schlingenendabschnitt (22) die keilförmige Schneide (40) bildet, und ein distaler Endbereich (34) des zweiten Strangs (30) mit einem dem Schlingenendabschnitt (22) entgegengesetzten Schlingenende (32) der Schlinge (18) verbunden ist.

5. Vorrichtung nach Anspruch 4, **dadurch gekennzeichnet, dass** der erste Strang (26) und zweite Strang (30) des Verbindungselements (16) gegenläufig schraubenlinienförmig gewunden sind.

6. Vorrichtung nach einem der Ansprüche 1 bis 5, **dadurch gekennzeichnet, dass** die Schlinge (18) etwa halbkreisförmig ausgebildet ist.

7. Vorrichtung nach einem der Ansprüche 1 bis 6, **dadurch gekennzeichnet, dass** die Schlinge (18) innenseitig und/oder außenseitig eine Schneidkante aufweist.

8. Vorrichtung nach einem der Ansprüche 1 bis 7, **dadurch gekennzeichnet, dass** das Verbindungselement (16) zumindest im Bereich des distalen Verbindungselementabschnitts (20) innenseitig und/oder außenseitig eine Schneidkante aufweist.

9. Vorrichtung nach einem der Ansprüche 1 bis 8, **dadurch gekennzeichnet, dass** der Schlingenträger (12) als Hohlrohr ausgebildet ist.

10. Resektoskop zum Abtragen von organischem Gewebe mittels Hochfrequenzstrom, mit einem Schaft (56), in dem eine mit Hochfrequenzspannung beaufschlagbare Vorrichtung (10) gemäß einem der Ansprüche 1 bis 9 angeordnet ist.

## Claims

1. A device for resection and/or ablation of organic tissue by means of high-freqency current, comprising a loop carrier (12), a loop (18) that can be acted upon by high-frequency voltage and is arranged at a distal end (14) of the loop carrier (12), and a connection-element (16) between the loop (18) and the loop carrier (12), wherein the loop carrier (12) can rotate about its longitudinal axis (36) in a direction of rotation, wherein a loop end portion (22) of the loop (18) and a distal connection-element portion (20) of the connection-element (16) form a wedge-shaped cutter (40), due to the fact that the loop end portion (22) and the distal connection-element portion (20) converge at an angle of less than 90°, and wherein the wedge-shaped cutter (40) points in the direction of rotation of the loop carrier (12), **characterized in that** the wedge-shaped cutter (40) points slightly in the distal direction so that the wedge-shaped cutter engages like a screw into the tissue due to the slight inclination in the distal direction.

2. The device of Claim 1, **characterized in that** the loop (18) extends in a plane transverse to a longitudinal axis (36) of the loop carrier (12).

3. The device of Claim 1 or 2, **characterized in that** the connection element (16) is designed in a helical line shape.

4. The device of anyone of Claims 1 through 3, **characterized in that** the connection element (16) has a first strand (26) and a second strand (30), a distal end area (24) of the first strand (26) forming the wedge-shaped cutter (40) together with the loop end portion (22), and a distal end area (34) of the second strand (30) being connected to an end (32) of the loop (18) opposite the loop end portion (22).

5. The device of Claim 4, **characterized in that** the first strand (26) and second strand (30) of the connection element (16) are wound in the shape of helical lines running in opposite directions.

6. The device of anyone of Claims 1 through 5, **characterized in that** the loop (18) has an approximately semicircular shape.

7. The device of anyone of Claims 1 through 6, **characterized in that** the loop (18) has a cutting edge on the inside and/or outside.

8. The device of anyone of Claims 1 through 7, **characterized in that** the connection element (16) has a cutting edge on the inside and/or outside at least in the area of the distal connection element portion (20).

9. The device of anyone of Claims 1 through 8, **characterized in that** the loop carrier (12) is designed as a hollow tube.

10. A resectoscope for removing organic tissue by means of high-frequency current, comprising a shaft (56) for receiving a device (10) that can be acted upon by high-frequency voltage and that is of the type according to anyone of Claims 1 through 9.

## Revendications

1. Dispositif de résection et/ou d'ablation de tissus organiques à l'aide d'un courant à haute fréquence, comportant un porte-boucle (12) comportant une boucle (18) alimentée par une tension à haute fréquence qui est disposée à une extrémité distale (14) du porte-boucle (12) et un élément de raccordement (16) entre la boucle (18) et le porte-boucle (12), le porte-boucle (12) pouvant tourner autour de son axe longitudinal (36) dans un sens de rotation, un segment terminal de boucle (22) de la boucle (18) et un segment de l'élément de raccordement (20) distal de l'élément de raccordement (16) formant une lame cunéiforme (40), le segment terminal de la boucle (22) et le segment d'élément de liaison distal (20) aboutissant l'un vers l'autre en formant un angle inférieur à 90° et la lame cunéiforme (40) étant orientée dans le sens de rotation du porte-boucle (12), **caractérisé en ce que** la lame cunéiforme (40) est légèrement orientée vers le plan distal de telle sorte que la boucle cunéiforme, compte tenu de sa légère inclinaison vers le plan distal, s'insère dans le tissu comme une vis.

2. Dispositif selon la revendication 1, **caractérisé en ce que** la boucle (18) s'étend dans un plan à peu près perpendiculaire par rapport à un axe longitudinal (36) du porte-boucle (12).

3. Dispositif selon la revendication 1 ou 2, **caractérisé en ce que** l'élément de liaison (16) est constitué en forme d'hélice.

4. Dispositif selon l'une des revendications 1 à 3, **caractérisé en ce que** l'élément de liaison (16) présente un premier brin (26) et un deuxième brin (30), une zone terminale distale (24) du premier brin (26) formant avec le segment terminal de la boucle (22) la lame cunéiforme (40) et une zone terminale distale (34) du deuxième brin (30) étant reliée à une extrémité de boucle (32) de la boucle (18) dans le sens opposé au segment terminal de la boucle (22).

5. Dispositif selon la revendication 4, **caractérisé en ce que** le premier brin (26) et le deuxième brin (30) de l'élément de liaison (16) sont enroulés de façon hélicoïdale en sens contraires.

6. Dispositif selon l'une des revendications 1 à 5, **caractérisé en ce que** la boucle (18) a une forme semi-circulaire.

7. Dispositif selon l'une des revendications 1 à 6, **caractérisé en ce que** la boucle (18) présente du côté interne et/ou du côté externe, une arête coupante.

8. Dispositif selon l'une des revendications 1 à 7, **caractérisé en ce que** l'élément de liaison (16) présente au moins dans la zone du segment distal de l'élément de liaison (20), une arête coupante du côté intérieur et/ou du côté extérieur.

9. Dispositif selon l'une des revendications 1 à 8, **caractérisé en ce que** le porte-boucle (12) est constitué comme un tube creux.

10. Résectoscope pour retirer des tissus organiques à l'aide d'un courant à haute fréquence, comportant une tige (56) dans laquelle est disposé un dispositif alimenté par une tension à haute fréquence (10) selon l'une des revendications 1 à 9.
